# FASCICULE DE BREVET EUROPEEN

(11) **EP 0 973 495 B1**
(45) Date de publication et mention de la délivrance du brevet: **16.01.2002**
(21) Numéro de dépôt: 98909556.7
(22) Date de dépôt: 17.02.1998
(51) Int. Cl.: A61K 7/48, A61K 7/06

(54) **UTILISATION D'EXTRAIT(S) DE GRAINES DE POIS BAMBARA DANS UNE COMPOSITION COSMETIQUE**
VERWENDUNG EINES BAMBARAERDNUSS-SAMENEXTRAKTES IN EINER KOSMETISCHEN ZUSAMENSETZUNG
USE OF BAMBARA NUT SEED EXTRACT(S) IN A COSMETIC COMPOSITION

(30) Priorité: 26.03.1997 FR 9703917
(43) Date de publication de la demande: 26.01.2000
(73) Titulaire: Cognis France S.A., 31360 Saint Martory (FR)
(72) Inventeur: PAULY, Gilles, F-54280 Seichamps (FR)
(74) Mandataire: Nuss, Pierre
(86) Numéro de dépôt international: FR9800306
(87) Numéro de publication internationale: WO9842305

(56) Documents cités:
- WO-A-93/23069
- WO-A-94/18944
- US-A- 5 489 426

## Description

La présent invention concerne le domaine de la cosmétologie et a pour objet l'utilisation cosmetique d'extraits de graines de pois Bambara (également appelé pois arachide ou haricot pistache) dans un produit ou une composition cosmétique, ainsi qu'un produit ou une composition cosmétique correspondant(e).

Le pois Bambara (Voandzeia subterranea (L) Thouars) est une graine d'origine africaine utilisée localement comme légume. II s'agit d'un légume indigène africain cultivé principalement par les fermiers comrne "culture de famine", un de ses principaux atouts étant sa tolérance à la sécheresse et aux sols pauvres et sa possibilité de croître dans des conditions inadaptées à l'arachide.

Les graines de pois Bambara qui constituent un aliment complet, contiennent des protéines, des hydrates de carbone et des lipides et elles peuvent être consommées aux différentes étapes de maturation.

Leur composition chimique (g/100g de farine ou pour 100g de graines sèches) est la suivante :
- protéines : 16 à 21 %,
- amidon : 39 à 49,5 %,
- tanins (équivalent acide tanique) : 0,36 à 0,94 %,
- lipides : 5 à 7,3 %,
- cendres : 3,65 %.

Il est connu que la graine de Voandzeia subterranea contient des inhibiteurs de protéases et l'activité de l'inhibiteur trypsique estimée par la technique dite de Kakadé serait d'après la littérature de 6,7 à 15,4 TUI/mg de farine, les propriétés fonctionnelles d'isolats protéiques de ladite graine ayant été analysés à des fins alimentaires.

W093/2 3069 divulgue une supplément de santé avec des phyto-estrogene des plantes, en outre de Voandzeia subterranea.

Or, les inventeurs ont constaté, de manière surprenante, que les extraits de graines de Voandzeia subterranea (pois Bambara), notamment les extraits protéiques, pouvaient être utilisés directement dans des produits ou des compositions cosmétiques et permettaient d'aboutir à des propriétés spécifiques et d'obtenir des effets particuliers marqués.

Ainsi, il a été constaté un fort pouvoir nutritif et stimulant cellulaire, un effet adoucissant et biofilmogène, des effets conditionneurs et réparateurs du relief cutané, des effets anti-rides, tenseurs, protecteurs dermiques et protecteurs du tissu élastique, ainsi que des effets anti-irritant, anti-radicaux libres, antipollution, hydratant, photo-protecteur anti-UVB et anti-UVA, apaisant, anti-protéases, anti-élastases, anti-collagénases, anti-catalase et anti-vieillissement et raffermissant cutané.

Des effets bio-conditionneur, réparateur, adoucissant et vitalisants ont été observés au niveau des cheveux et des ongles après application des extraits précités.

Le premier objet de la présente invention consiste, par conséquent, en l'utilisation d'au moins une fraction protéique soluble extraite de la graine de pois Bambara (Voandzeia subterranea) en tant qu'agent actif dans un produit ou une composition cosmétique pour la peau et/ou les phanères.

Les extraits précités peuvent être utilisés, non seulement pour des applications de soins et d'hygiène de la peau (produits pour le visage et le corps, produits de jour ou de nuit, produits solaires, produit hygiène; anti-rides, ou produits amincissants), mais également dans le domaine des soins et de l'hygiène capillaires (lotions ou shampooings, crèmes, mousses, produits protecteurs, réparateurs, adoucissants, filmogènes et photo protecteurs ou encore produits pour permanente et de coloration).

La préparation des extraits protéiques solubles est réalisée par les techniques conventionnelles d'extraction des protéines végétales et de préparation des concentrés ou isolats protéiques, connus de l'homme du métier.

A titre d'exemples illustratifs non limitatifs, on décrira ci-après différents procédés d'obtention et de préparation d'extraits de graines de pois Bambara, susceptibles d'être utilisés dans le cadre de la présente invention.

### Exemple 1 :

On ajoute dans 2,5 litres d'eau distillée, 250 g de farine obtenue par broyage de graines de Voandzeia subterranea.

Après 15 minutes d'agitation, le pH de la solution est ajusté à 7,5 avec de la soude et l'extraction est menée pendant une heure à température ambiante.

Après centrifugation pendant 10 minutes à 5000 g, on élimine la couche lipidique supérieure : le surnageant beige est recueilli, puis filtré sur 0,5 µ.

La solution comprend 2,5 % d'extrait sec et présente une concentration en protéines de 12 g/l (dosage du Biuret).

L'extrait peut être déshydraté par les techniques traditionnelles connues, telles que l'atomisation, la lyophilisation ou analogue.

L'activité antitrypsique de l'atomisat (déterminée par la technique dite de Kakadé) est de : 33,6 TUI/mg, soit une activité de la solution en se basant sur l'extrait sec de 840 TUI/ml.

### Exemple 2 :

On ajoute dans 4 litres d'eau distillée 400 g de farine obtenue par broyage de graines de Voandzeia subterranea, et on traite la solution comme dans l'exemple 1.

On obtient 3,2 litres d'une solution peu colorée comprenant 3 % d'extrait sec et présentant une concentration en protéines de 17,8 g/l.

Le pH de la solution est ajusté à 4,5 avec de l'acide sulfurique, chlorhydrique ou phosphorique, puis mis sous agitation pendant 30 minutes.

La solution est ensuite centrifugée pendant 15 minutes à 5000 g et le précipité et le surnageant sont ensuite recueillis.

Le précipité est mis en solution dans un volume d'eau correspondant à 10 % du volume avant précipitation et le pH de la solution obtenue est ajusté avec NaOH jusqu'à stabilisation à 7,5.

La solution est ensuite de nouveau centrifugée pour éliminer l'insoluble, et on obtient finalement une solution à 9,7 % d'extrait sec, que l'on déshydrate par atomisation.

L'activité antitrypsique de l'atomisat est de 11,2 TUI/mg, soit une activité de la solution en se basant sur l'extrait sec de 1087 TUI/ml.

### Exemple 3 :

Le surnageant obtenu après précipitation des protéines selon l'exemple 2 est filtré sur 0,22 µ et on obtient une solution claire comprenant 1,41 % d'extrait sec et de concentration en protéines de 1,68 g/l.

La solution est ensuite déshydratée par atomisation.

L'activité antitrypsique de l'atomisat est de 46,6 TUI/mg, soit une activité théorique de la solution en se basant sur l'extrait sec de 657 TUI/ml.

L'analyse en perméation de gel sur colonne du type superose 12HR des fractions extraites par les différents procédés précités, permet de caractériser au moins huit fractions plus ou moins importantes selon les extraits.

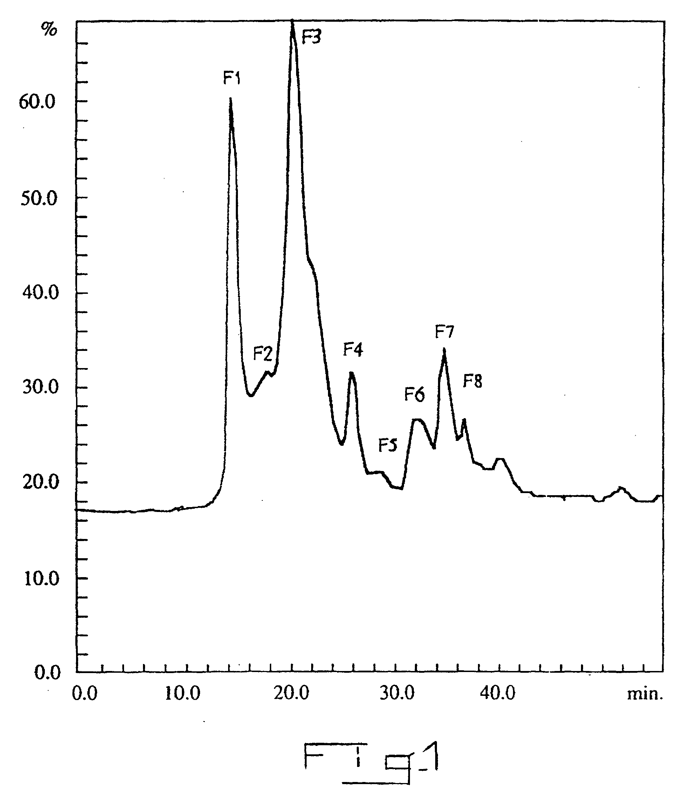

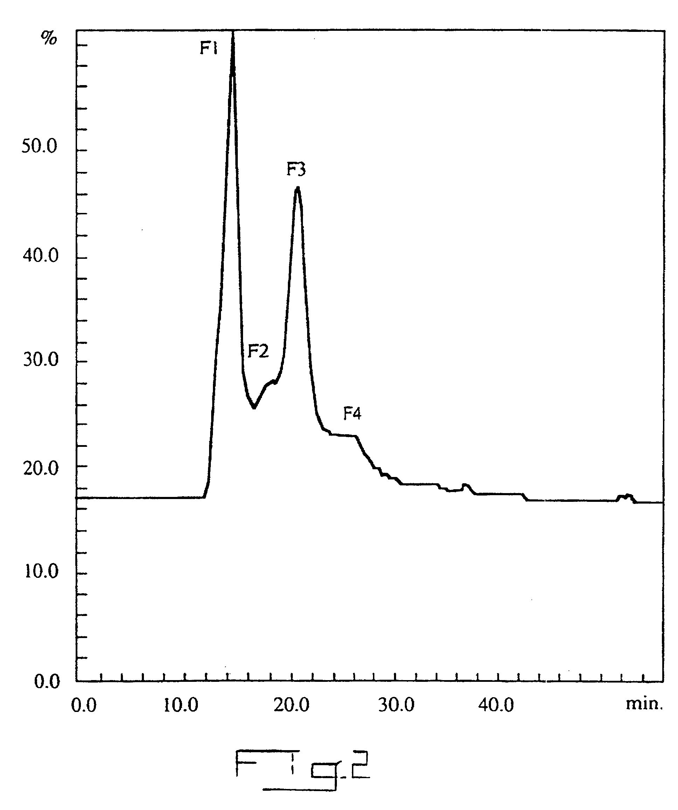

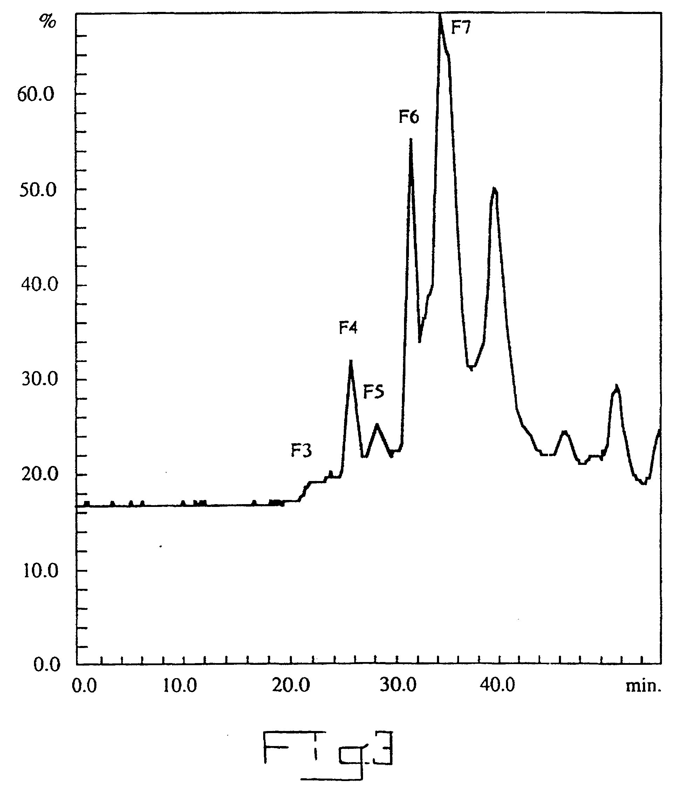

Les figures 1 à 3 des dessins annexés représentent, respectivement, sous forme de chromatogrammes, les profils des poids moléculaires des extraits obtenus successivement par chacun des exemples de procédés 1 à 3 précités (Figure 1 = Exemple 1 ; Figure 2 = Exemple 2 ; Figure 3 = Exemple 3).

Sur ces figures, on peut relever :
- une fraction (notée F1) de très haut poids moléculaire (entre 1 000 000 et 1 200 000 Da d'après l'étalonnage de la colonne),
- une fraction F2 de poids moléculaire compris entre 400 000 et 500 000 Da,
- une fraction F3 de poids moléculaire compris entre 150 000 et 180 000 Da,
- une fraction F4 de poids moléculaire compris entre 30 000 et 35 000 Da,
- une fraction F5 de poids moléculaire compris entre 15 000 et 18 000 Da,
- une fraction F6 de poids moléculaire compris entre 4 800 et 5 500 Da,
- une fraction F7 de poids moléculaire compris entre 2 100 et 2 500 Da,
- une fraction F8 de poids moléculaire compris entre 1 000 et 1 300 Da.

Les extraits obtenus au moyen des exemples de procédés décrits ci-dessus sont directement utilisables sous forme liquide, ou après séchage selon les techniques conventionnelles de déshydratation (atomisation, lyophilisation ou analogue).

Les fractions protéiques peuvent être utilisées soit sous leur forme native, sans modification de leurs structures, soit sous la forme d'association(s) naturelle(s) de deux ou de toutes les fractions extraites correspondant aux différents pics des chromatogrammes représentés sur les dessins annexés, ou encore sous forme isolée.

Elles peuvent également être utilisées sous différentes formes galéniques, telles que solutés, liposomes, nanosphères, microsphères, microcapsules, micelles ou analogues.

En variante, ces fractions protéiques peuvent être utilisées sous leur forme modifiée ou fonctionnalisée par l'intermédiaire de l'un quelconque des traitements suivants :
- polymérisation,
- hydrolyse chimique des protéines,
- hydrolyse enzymatique des protéines par des protéases d'origine animale, végétale, microbienne ou fongique : pepsine, trypsine, chymotrypsine, papaine, pronase, bromelaine, endoprotéinase, thermitase ou protéases de bacillus subtilis, d'Aspergillus niger et d'Aspergillus oryzae (subtilisine, alcalase, neutrase),
- transformation microbienne, avec utilisation des protéines de pois Bambara en tant que substrat de fermentation, par divers micro-organismes tels que des levures (Saccharomyces), des moisissures (Aspergillus) ou des bactéries (Bacillus et analogues),
- fonctionnalisation chimique ou enzymatique par des procédés tels que le désamidation, succinilation ou phosphorylation,
- quaternisation,
- greffage de molécules saccharidiques ou lipidiques.

La présente invention a également pour objet une composition cosmétique, notamment à usage topique pour la peau et/ou les phanères, caractérisée en ce qu'elle contient au moins une fraction protéique soluble extraite de la graine de pois Bambara (Voandzeia subterranea).

La ou les fraction(s) protéique(s) précitée(s) est (sont) extraite(s) par l'eau ou une solution aqueuse, notamment des solutions salines à différents pH, le cas échéant au moyen d'un générateur à ultrasons et est (sont) purifiée(s) par précipitation (modification de pH, sels, solvants, variation de températures), par adsorption, par chromatographie d'échange d'ions ou d'affinité ou par ultrafiltration.

De manière avantageuse, ladite composition cosmétique contient au moins une fraction protéique extraite de graines de pois Bambara et enrichie en inhibiteurs de protéases, la ou les fraction(s) protéique(s) présente(s) consistant en au moins une fraction protéique totale ou native présentant, en filtration sur gel, un poids moléculaire apparent de 1 000 000 à 1 200 000 Da, de 400 000 à 500 000 Da, de 150 000 à 180 000 Da, de 30 000 à 35 000 Da, de 15 000 à 18 000 Da, de 4800 à 5500 Da, de 2100 à 2500 Da ou de 1000 à 1300 Da.

La ou les fraction(s) protéique(s) peuvent soit consister en un hydrolysat chimique ou enzymatique préparé à partir de protéines natives, soit être obtenue(s) par polymérisation de protéines natives, ou encore être chimiquement modifiées (quaternisation, succinilation, greffage de polysaccharides, lipides ou autres).

Conformément à une première variante de réalisation de l'invention, la composition cosmétique contient au moins deux fractions protéiques solubles extraites de graines de pois Bambara de poids moléculaires apparents différents.

Selon une seconde variante de réalisation de l'invention, la composition cosmétique contient un extrait de graines de Voandzeia subterranea, constitué par l'ensemble des fractions protéiques extractibles présentes naturellement dans la graine.

Préférentiellement, ladite composition cosmétique comporte entre 0,01 % et 50,00 %, préférentiellement entre 0,50 % et 15,00 %, en poids de fraction(s) protéique(s) extraite(s) de graines de Voandzeia subterranea.

A titre d'exemples non limitatifs de réalisations pratiques de l'invention, on décrira ci-après différents produits ou compositions cosmétiques comprenant au moins un extrait protéique de graines de pois Bambara.

### Exemple 1:

Un produit cosmétique sous forme de crème protectrice, adoucissante et calmante pour le visage conforme à l'invention pourra, par exemple, présenter une composition pondérale, constituée à partir des fractions A et B suivantes, telle qu'indiquée ci-après.

### Fraction A (phase grasse) :

- Cire d'abeilles 9,00 %
- Octyldodécanol 12,00 %
- Oléate décylique 10,00 %
- Huile de sésame 12,50 %
- Vaseline 10,00%
- Myristate d'isopropyle 2,00 %
- Alcool cétylique 0,80 %
- Cholestérol 0,30 %
- Palmitate cétylique 2,50 %

### Fraction B (phase aqueuse) :

- Sorbitol 4,00 %
- Borate de sodium 0,30 %
- Extrait aqueux de Voandzeia subterranea préparé selon l'exemple 3, (forme hydrosolutée) 5,00 %
- Conservateur LS388 (Laboratoires Sérobiologiques) 2,00 %
- Eau qsp 100,00 %

Le procédé de préparation et de fabrication de la crème pour le visage susvisée consiste essentiellement à préparer séparément la phase grasse à 80° C et la phase aqueuse à 75° C (sans l'extrait de Voandzeia subterranea), puis à verser la phase aqueuse dans la phase grasse sous agitation turbine, à laisser refroidir et, vers 50° C, à ajouter l'extrait de Voandzeia subterranea et enfin à agiter jusqu'à refroidissement à température ambiante.

### Exemple 2 :

Un produit cosmétique sous forme de crème de jour anti-irritante, photoprotectrice, raffermissante et hydratante conforme à l'invention pourra, par exemple, présenter une composition pondérale, constituée à partir des fractions A et B suivantes, telle qu'indiquée ci-après.

### Fraction A (phase grasse) :

- Stéarate de glycérol SE 12,00 %
- Alcool cétylique 1,00 %
- Glycérides de coco hydrogénés 2,00 %
- Huile de jojoba 3,00 %

### Fraction B (phase aqueuse) :

- Glycérine 5,00 %
- Extrait lyophilisé de Voandzeia subterranea préparé selon l'exemple 1 1,00 %
- Elestab 4112 (Laboratoires Sérobiologiques) 0,40 %
- Parfum 0,30 %
- Eau qsp 100,00 %

Le procédé de préparation et de fabrication de la crème de jour susvisée consiste essentiellement à préparer séparément la phase grasse à 80° C et la phase aqueuse à 80° C (sans l'extrait de Voandzeia subterranea), puis à verser la phase grasse dans la phase aqueuse sous agitation turbine, à laisser refroidir et, vers 45° C, à ajouter l'extrait lyophilisé de Voandzeia subterranea, préalablement dissout dans trois fois son poids en eau, et enfin à poursuivre l'agitation jusqu'à refroidissement à température ambiante.

### Exemple 3 :

Un produit cosmétique sous forme de lait pour le corps à propriétés hydratante, adoucissante, apaisante, défatigante et renforçatrice d'élasticité conforme à l'invention pourra, par exemple, présenter une composition pondérale, constituée à partir des fractions A et B suivantes, telle qu'indiquée ci-après.

### Fraction A (phase grasse) :

- Stéarate de glycérol (et) Stéarate de PEG 40 6,00 %
- Palmitate d'isopropyle 8,50 %
- Huile de paraffine 5,00 %
- Diméthicone 0,50 %
- Alcool cétylique 1,00 %

### Fraction B (phase aqueuse) :

- Glycérine 2,00 %
- Elestab 4112 (Laboratoires Sérobiologiques) 0,40 %
- Extrait aqueux de Voandzeia subterranea préparé selon l'exemple 2 (sous forme d'atomisat) 0,50 %
- Eau qsp 100,00 %

Le procédé de préparation et de fabrication du lait pour le corps susvisé est sensiblement identique à celui du produit cosmétique de l'exemple 2.

### Exemple 4 :

Un produit cosmétique sous forme de crème de nuit à propriétés anti-âge, anti-rides, réparatrice, protectrice et nourrissante conforme à l'invention pourra, par exemple, présenter une composition pondérale, constituée à partir des fractions A et B suivantes, telle qu'indiquée ci-après.

### Fraction A (phase grasse) :

- Stéarate de glycérol (et) Alcool cétostéarylique (et) Palmitate cétylique (et) Glycérides de coco 10,00 %
- Stéarate de glycérol PEG 20 2,00 %
- Cétéareth 12 1,00 %
- Oléate décylique 6,00 %
- Stéarate d'octyle 6,00 %
- Elestab 4121 (Laboratoires Sérobiologiques) 0,20 %

### Fraction B (phase aqueuse) :

- Glycérine 4,00 %
- Extrait aqueux de Voandzeia subterranea préparé selon l'exemple 2 (sous forme de soluté) 10,00 %
- Elestab 305 (Laboratoires Sérobiologiques) 0,50 %
- Parfum 0,20 %
- Eau qsp 100,00 %

Le procédé de préparation et de fabrication de la crème de nuit susvisée est sensiblement identique à celui du produit cosmétique de l'exemple 2.

## Revendications

1. Utilisation cosmetique d'au moins une fraction protéique soluble extraite de la graine de pois Bambara (Voandzeia subterranea) en tant qu'agent actif dans un produit ou une composition cosmétique pour la peau et/ou les phanères.

2. Composition cosmétique, notamment à usage topique pour la peau et/ou les phanères, **caractérisée en ce qu'**elle contient au moins une fraction protéique soluble extraite de la graine de pois Bambara (Voandzeia subterranea).

3. Composition cosmétique selon la revendication 2, **caractérisée en ce que** la ou les fraction(s) protéique(s) est (sont) extraite(s) par l'eau ou une solution aqueuse, notamment, des solutions salines à différents pH, le cas échéant au moyen d'un générateur à ultrasons.

4. Composition cosmétique selon l'une quelconque des revendications 2 et 3, **caractérisée en ce que** la ou les fraction(s) protéique(s) extraite(s) est (sont) purifiée(s) par précipitation, par adsorption, par chromatographie d'échange d'ions ou d'affinité ou par ultrafiltration.

5. Composition cosmétique selon l'une quelconque des revendications 2 à 4, **caractérisée en ce qu'**elle contient au moins une fraction protéique extraite de graines de pois Bambara et enrichie en inhibiteurs de protéases.

6. Composition cosmétique selon l'une quelconque des revendications 2 à 5, **caractérisée en ce que** la ou les fractions protéique(s) présente(s) consiste(nt) en au moins une fraction protéique totale ou native présentant, en filtration sur gel, un poids moléculaire apparent de 1 000 000 à 1 200 000 Da, de 400 000 à 500 000 Da, de 150 000 à 180 000 Da, de 30 000 à 35 000 Da, de 15 000 à 18 000 Da, de 4800 à 5500 Da, de 2100 à 2500 Da ou de 1000 à 1300 Da.

7. Composition cosmétique selon l'une quelconque des revendications 2 à 6, **caractérisée en ce que** la ou les fraction(s) protéique(s) consiste(nt) en un hydrolysat chimique ou enzymatique préparé à partir de protéines natives.

8. Composition cosmétique selon l'une quelconque des revendications 2 à 7, **caractérisée en ce que** la ou les fraction(s) protéique(s) est sont obtenue(s) par polymérisation de protéines natives.

9. Composition cosmétique selon l'une quelconque des revendications 2 à 8, **caractérisée en ce que** la ou les fraction(s) protéique(s) est (sont) chimiquement modifiées par greffage.

10. Composition cosmétique selon l'une quelconque des revendications 6 à 9, **caractérisée en ce qu'**elle contient au moins deux fractions protéiques solubles extraites de graines de pois Bambara de poids moléculaires apparents différents.

11. Composition cosmétique selon l'une quelconque des revendications 2 à 5 et 7 à 9, **caractérisée en ce qu'**elle contient un extrait de graines de Voandzeia subterranea, constitué par l'ensemble des fractions protéiques extractibles présentes naturellement dans la graine.

12. Composition cosmétique selon l'une quelconque des revendications 2 à 11, **caractérisée en ce qu'**elle comporte entre 0,01 % et 50,00 %, préférentiellement entre 0,50 % et 15,00 %, en poids de fraction(s) protéique(s) extraite(s) de graines de Voandzeia subterranea.

## Claims

1. Cosmetic use of at least one soluble protein fraction extracted from the seed of Bambara (Voandzeia subterranea) nut as an active agent in a cosmetic product or composition for the skin and/or the hair, nails and eyelashes.

2. Cosmetic composition, particularly for topical use for the skin and/or the hair, nails and eyelashes, **characterized in that** it contains at least one soluble protein fraction extracted from the seed of Bambara (Voandzeia subterranea) nut.

3. Cosmetic composition according to claim 2, **characterized in that** the protein fraction(s) is extracted with water or an aqueous solution, particularly saline solutions of different pH, as the case may be, by means of an ultrasonic generator.

4. Cosmetic composition according to any one of claims 2 and 3, **characterized in that** the extracted protein fraction(s) is purified by precipitation, by adsorption, by chromatographic ion exchange or affinity or by ultrafiltration.

5. Cosmetic composition according to any one of claims 2 to 4, **characterized in that** it contains at least one protein fraction extracted from seed of Bambara nut and enriched in protease inhibitors.

6. Cosmetic composition according to any one of claims 2 to 5, **characterized in that** the protein fraction(s) present consists of at least one total or native protein fraction having, by gel filtration, an apparent molecular weight of-1,000,000 to 1,200,000 Da, from 400,000 to 500,000 Da, from 150,000 to 180,000 Da, from 30,000 to 35,000 Da, from 15,000 to 18,000 Da, from 4,800 to 5,550 Da, from 2,100 to 2,500 Da or from 1,000 to 1,300 Da.

7. Cosmetic composition according to any one of claims 2 to 6, **characterized in that** the protein fraction(s) consists of a chemical or enzymatic hydrolyzate prepared from native proteins.

8. Cosmetic composition according to any one of claims 2 to 7, **characterized in that** the protein fraction(s) is obtained by polymerization of native proteins.

9. Cosmetic composition according to any one of claims 2 to 8, **characterized in that** the protein fraction(s) is chemically modified by grafting.

10. Cosmetic composition according to any one of claims 6 to 9, **characterized in that** it contains at least two soluble protein fractions extracted from seed of Bambara nut of different apparent molecular weights.

11. Cosmetic composition according to any one of claims 2 to 5 and 7 to 9, **characterized in that** it contains an extract of seed of Voandzeia subterranea, constituted by all the extractable protein fractions present naturally in the seed.

12. Cosmetic composition according to any one of claims 2 to 11, **characterized in that** it contains between 0.01% and 50.00%, preferably between 0.50% and 15.00%, by weight of protein fraction(s) extracted from seed of Voandzeia subterranea.

## Patentansprüche

1. Kosmetische Verwendung mindestens einer aus dem Samen der Bambaranuß (Voandzeia subterranea) extrahierten löslichen Proteinfraktion als Wirkstoff in einem Kosmetikprodukt bzw. einer Kosmetikzusammensetzung für die Haut und/oder die Epithelanhanggebilde.

2. Kosmetikzusammensetzung, insbesondere zur topischen Anwendung auf die Haut und/oder die Epithelanhanggebilde, **dadurch gekennzeichnet, daß** sie mindestens eine aus Bambaranuß-(Voandzeia subterranea) Samen extrahierte lösliche Proteinfraktion enthält.

3. Kosmetikzusammensetzung nach Anspruch 2, **dadurch gekennzeichnet, daß** die Proteinfraktion(en) mit Wasser oder einer wäßrigen Lösung, insbesondere Salzlösungen mit unterschiedlichen pH-Werten, gegebenenfalls mittels eines Ultraschallgeräts, extrahiert wird bzw. werden.

4. Kosmetikzusammensetzung nach Anspruch 2 oder 3, **dadurch gekennzeichnet, daß** die extrahierte Proteinfraktion bzw. extrahierten Proteinfraktionen durch Fällung, Adsorption, Ionenaustauschchromatographie, Affinitätschromatographie oder Ultrafiltration gereinigt wird bzw. werden.

5. Kosmetikzusammensetzung nach einem der Ansprüche 2 bis 4, **dadurch gekennzeichnet, daß** sie mindestens eine aus Bambaranußsamen extrahierte Proteinfraktion, die mit Proteaseinhibitoren angereichert ist, enthält.

6. Kosmetikzusammensetzung nach einem der Ansprüche 2 bis 5, **dadurch gekennzeichnet, daß** die vorliegende Proteinfraktion bzw. die vorliegenden Proteinfraktionen aus mindestens einer Gesamtproteinfraktion oder nativen Proteinfraktion besteht bzw. bestehen, die bei Gelfiltration ein scheinbares Molekulargewicht von 1 000 000 bis 1 200 000 Da, 400 000 bis 500 000 Da, 150 000 bis 180 000 Da, 30 000 bis 35 000 Da, 15 000 bis 18 000 Da, 4 800 bis 5 500 Da, 2 100 bis 2 500 Da oder 1 000 bis 1 300 Da aufweist.

7. Kosmetikzusammensetzung nach einem der Ansprüche 2 bis 6, **dadurch gekennzeichnet, daß** die Proteinfraktion(en) aus einem chemischen oder enzymatischen Hydrolysat von nativen Proteinen besteht bzw. bestehen.

8. Kosmetikzusammensetzung nach einem der Ansprüche 2 bis 7, **dadurch gekennzeichnet, daß** die Proteinfraktion(en) durch Polymerisation von nativen Proteinen erhalten wird bzw. werden.

9. Kosmetikzusammensetzung nach einem der Ansprüche 2 bis 8, **dadurch gekennzeichnet, daß** die Proteinfraktion(en) chemisch durch Pfropfung modifiziert ist bzw. sind.

10. Kosmetikzusammensetzung nach einem der Ansprüche 6 bis 9, **dadurch gekennzeichnet, daß** sie mindestens zwei aus Bambaranußsamen extrahierte lösliche Proteinfraktionen mit unterschiedlichen scheinbaren Molekulargewichten enthält.

11. Kosmetikzusammensetzung nach einem der Ansprüche 2 bis 5 sowie 7 bis 9, **dadurch gekennzeichnet, daß** sie einen aus allen natürlich im Samen vorkommenden extrahierbaren Proteinfraktionen bestehenden Voandzeia-subterranea-Samenextrakt enthält.

12. Kosmetikzusammensetzung nach einem der Ansprüche 2 bis 11, **dadurch gekennzeichnet, daß** sie 0,01 Gew.-% bis 50,00 Gew.-%, vorzugsweise 0,50 Gew.-% bis 15,00 Gew.-% einer aus Voandzeia-subterranea-Samen extrahierten Proteinfraktion bzw. von aus Voandzeiasubterranea-Samen extrahierten Proteinfraktionen enthält.
